# EUROPEAN PATENT SPECIFICATION

(11) **EP 2 488 482 B1**
(45) Date of publication and mention of the grant of the patent: **19.08.2015**
(21) Application number: 10739810.9
(22) Date of filing: 30.07.2010
(51) Int. Cl.: C07C 209/26, C07C 209/48, C07C 211/09, C07C 211/18

(54) **PROCESS FOR IMPROVING THE CATALYTIC ACTIVITY OF CATALYST SYSTEMS FOR REDUCTIVE AMINATION OF ALIPHATIC CYANOALDEHYDES TO ALIPHATIC DIAMINES**
VERFAHREN ZUR VERBESSERUNG DER KATALYTISCHEN WIRKUNG VON KATALYSATORSYSTEMEN FÜR DIE REDUKTIVE AMINIERUNG VON ALIPHTISCHEN CYANOALDEHYDEN ZU ALIPHATISCHEN DIAMINEN
PROCÉDÉ DESTINÉ À AMÉLIORER L'ACTIVITÉ CATALYTIQUE DE SYSTÈMES CATALYSEURS POUR L'AMINATION RÉDUCTRICE DE CYANO-ALDÉHYDES ALIPHATIQUES EN DIAMINES ALIPHATIQUES

(30) Priority: 31.07.2009 US 230362 P
(43) Date of publication of application: 22.08.2012
(73) Proprietor: Dow Global Technologies LLC, Midland, MI 48674 (US)
(72) Inventor: HICKMAN, Daniel, Midland MI 48642 (US); FEIST, Shawn, Midland MI 48642 (US); MOLITOR, Erich, Midland MI 48640 (US); MOLZAHN, David, Midland MI 48642 (US); SANTHANY, Stacie, Auburn MI 48611 (US); SCHUITMAN, Abraham, Midland MI 48642 (US)
(74) Representative: Boult Wade Tennant
(86) International application number: PCT/US2010/043899
(87) International publication number: WO 2012/087266

(56) References cited:
- DE-A1-102006 006 625
- DE-C1- 19 614 154
- GOMEZ S ET AL: "The Reductive Amination of Aldehydes and Ketones and the Hydrogenation of Nitriles: Mechanistic Aspects and Selectivity Control", ADVANCED SYNTHESIS & CATALYSIS, WILEY VCH VERLAG, WEINHEIM, DE, vol. 344, no. 10, 1 January 2002 (2002-01-01), pages 1037-1057, XP002420447, ISSN: 1615-4150, DOI: DOI:10.1002/1615-4169(200212)344:10&LT,103 7::AID-ADSC1037&GT,3.0.CO,2-3

## Description

### Field of Invention

The instant invention relates to a process for improving catalytic activity of catalyst systems for reductive amination of aliphatic cyanoaldehydes to aliphatic diamines.

### Background of the Invention

Bis(aminomethyl)cyclohexane is a diamine that has applications as a precursor to an aliphatic diisocyanate (bis(isocyanatomethyl)cyclohexane). It is useful as a chain extender in certain polyurethanes systems and can be used as an epoxy curing agent. Bis(aminomethyl)cyclohexane exists as a number of isomers, of which the 1,3- and 1,4-isomers are of primary interest. The 1,3-and 1,4-isomers can also exist in a number of diastereomeric forms, as the aminomethyl groups can each reside above or below the plane of the cyclohexane ring.

1,3- and 1,4-bis(aminomethyl)cyclohexane mixtures can be prepared via a number of synthetic routes. U. S. Patent No. 3,143,570 describes a two-step process that requires preparation and isolation of the intermediate solid iminomethylcyclohexanecarbonitriles in water.

As another example, a route may start with butadiene and acrolein, forming 1,2,3,6-tetrahydrobenzaldehyde in a Diels-Alder reaction. This intermediate is then hydroformylated to add a second aldehyde group and reductively aminated to form the desired diamine. A mixture of isomeric forms of the diamine is obtained, as for example, described in the U. S. Patent No. 6,252,121.

The reductive amination of hydroformylated 1,2,3,6-tetrahydrobenzaldehyde using a sponge-metal catalyst or nickel on silica gel/alumina as in U. S. Patent No. 6,252,121, however, tends to produce diamine products in low yields. A significant portion of the starting material forms unwanted by-products and polymeric species. As a result, raw material costs may be high and purification of the crude product can be difficult and expensive. Polymeric by-products often foul the reactor and downstream purification unit operations.

It is sometimes possible to suppress by-product formation in reductive amination reactions by "protecting" (or "blocking") the aldehyde groups with an alkyl amine as, for example, described in the U. S. Patent Nos. 5,041,675 and 5,055,618. The blocked groups are more resistant to polymerization and other unwanted side reactions. However, this approach requires the use of additional raw materials and introduces additional chemical species into the reaction, which must later be removed from the crude product and recycled. Process yields are still far short of those that are needed to have a highly economical process.

Additionally, the production of 1,3- and 1,4-bis(aminomethyl)cyclohexane via a dialdehyde intermediate may be difficult due to catalyst deactivation that leads to rapidly declining yields. Although more stable catalysts have been identified, these catalysts provide lower yields from the very beginning of operation. In addition, the dialdehyde intermediate route requires a reliable and sufficient supply of acrolein.

DE 10 2006 006 625 A1 discloses a method of producing amines in which a catalyst is treated with ammonia before the hydrogenation or reductive amination process.

In order to overcome these catalyst performance issues and avoid potential future acrolein supply issues, the instant invention provides reductive amination of 1,3- and 1,4-cyanocyclohexane carboxaldehyde (CCA). This intermediate is based on an acrylonitrile feedstock, which is more accessible than acrolein. Simultaneous reduction of the nitrile group and reductive amination of the aldehyde functionality require a specialized catalyst. Traditional nitrile reduction conditions and catalysts are more aggressive than aldehyde reductive amination conditions and catalysts. Thus, catalysts and conditions that are effective for complete reduction of the nitrile group may also have a tendency to reduce the aldehyde to the corresponding alcohol, resulting in a yield loss. On the other hand, catalysts and conditions that are typically chosen for reductive amination of an aldehyde are typically ineffective in providing complete reduction of the nitrile group, resulting in yield losses to the intermediate aminonitriles. Additionally, the relatively short lifetime of current catalysts introduces other challenges. Catalysts providing good yields to the diamine product (1,3- and 1,4-bis(aminomethyl)cyclohexanes) consistently lose their activity for the nitrile hydrogenation step within less than 250 hours of time on stream. Economically viable catalysts for this process require a much higher number of hours of lifetime, or, equivalently, pounds of 1,3- and 1,4-bis(aminomethyl)cyclohexanes produced per pound of catalyst.

Accordingly, it would be desirable to provide a method by which cycloaliphatic bis(aminomethyl) compounds can be prepared economically and in high yield. Furthermore, it would be desirable to provide a process for improving catalytic activity of catalyst systems for reductive amination of aliphatic cyanoaldehydes to aliphatic diamines.

### Summary of the Invention

The instant invention provides a process for improving catalytic activity of catalyst systems for reductive amination of aliphatic cyanoaldehydes to aliphatic diamines. Pressures were measured in psi or psig(1 psi or psig ≃ 68.95 mbar).

In one embodiment, the instant invention provides a process for improving catalytic activity of catalyst systems for reductive amination of aliphatic cyanoaldehydes to aliphatic diamines comprising the steps of: (1) feeding ammonia, optionally hydrogen, and one or more solvents over one or more heterogeneous metal based catalyst systems having a reduced catalytic activity for a period of greater than 1 hour at a temperature in the range of from 50° C to 500° C; wherein said one or more heterogeneous metal based catalyst systems have a yield of less than 90 percent based on the molar conversion of cyanoaldehydes to diamines; (2) subsequently feeding hydrogen and optionally one or more solvents over said one or more heterogeneous metal based catalyst systems for a period of greater than 1 hour at a temperature in the range of from 100 ° C to 500° C; and (3) thereby improving the catalytic activity of said one or more heterogeneous metal based catalyst systems.

In another embodiment, the instant invention further provides one or more heterogeneous metal based catalyst systems having improved catalytic activity obtained via the preceding method.

Further described is a process for improving catalytic activity of catalyst systems for reductive amination of aliphatic cyanoaldehydes to aliphatic diamines comprising the steps of: (1) feeding ammonia, optionally hydrogen, and optionally one or more solvents over one or more heterogeneous metal based catalyst systems having a reduced catalytic activity for a period of greater than 1 hour at a temperature in the range of from 50° C to 500° C; wherein said one or more heterogeneous metal based catalyst systems have a yield of less than 90 percent based on the molar conversion of cyanoaldehydes to diamines; and (2) thereby improving the catalytic activity of said one or more heterogeneous metal based catalyst systems.

Further described is a process for improving catalytic activity of catalyst systems for reductive amination of aliphatic cyanoaldehydes to aliphatic diamines comprising the steps of: (1) feeding hydrogen and optionally one or more solvents over one or more heterogeneous metal based catalyst systems having a reduced catalytic activity for a period of greater than 1 hour at a temperature in the range of from 100° C to 500° C; wherein said one or more heterogeneous metal based catalyst systems have a yield of less than 90 percent based on the molar conversion of cyanoaldehydes to diamines; and (2) thereby improving the catalytic activity of said one or more heterogeneous metal based catalyst systems.

Further described are cycloaliphatic diamines comprising the reaction product of one or more cycloaliphatic cyanoaldehydes selected from the group consisting of 1,3-cyanocyclohexane carboxaldehyde, 1,4-cyanocyclohexane carboxaldehyde, mixtures thereof, and combinations thereof, hydrogen, and ammonia fed into a reductive amination reactor system; wherein said one or more cycloaliphatic cyanoaldehydes, hydrogen, and ammonia are contacted with each other in the presence of one or more heterogeneous metal based catalyst systems at a temperature in the range of from 80° C to about 160° C and a pressure in the range of from 700 to 3500 psig; wherein said one or more cycloaliphatic diamines are selected from the group consisting of 1,3-bis(aminomethyl)cyclohexane, 1,4-bis(aminoniethyl)cyclohexane, combinations thereof, and mixtures thereof.

Further described is a process for producing cycloaliphatic diamines comprising the steps of: (1) feeding one or more cycloaliphatic cyanoaldehydes selected from the group consisting of 1,3-cyanocyclohexane carboxaldehyde, 1,4-cyanocyclohexane carboxaldehyde, mixtures thereof, and combinations thereof, hydrogen, and ammonia into a reductive amination reactor system; (2) contacting said one or more cycloaliphatic cyanoaldehydes, hydrogen, and ammonia with each other in the presence of one or more heterogeneous metal based catalyst systems at a temperature in the range of from 80° C to about 160° C and a pressure in the range of from 700 to 3500 psig; and (3) thereby forming one or more cycloaliphatic diamines, wherein said one or more cycloaliphatic diamines are diamines selected from the group consisting of 1,3-bis(aminomethyl)cyclohexane, 1,4-bis(aminomethyl)cyclohexane, combinations thereof, and mixtures thereof.

### Brief Description of the Drawings

For the purpose of illustrating the invention, there is shown in the drawings a form that is exemplary; it being understood, however, that this invention is not limited to the precise arrangements and instrumentalities shown.
Fig. 1 is a graph illustrating the relationship between diamine product yield and time on stream (TOS) as well as illustrating the relationship between the amount of diamine product yield in grams and the amount of catalyst in grams in the reactor of the Inventive Example 1; and
Fig. 2 is a graph illustrating the relationship between diamine product yield and time on stream (TOS) as well as illustrating the relationship between the amount of diamine product yield in grams and the amount of catalyst in grams in the reactor of the Inventive Example 2.

### Detailed Description of the Invention

The instant invention provides a process for improving catalytic activity of catalyst systems for reductive amination of aliphatic cyanoaldehydes to aliphatic diamines.

Further described is a process for improving catalytic activity of catalyst systems for reductive amination of aliphatic cyanoaldehydes to aliphatic diamines comprising the steps of: (1) feeding ammonia, optionally hydrogen, and optionally one or more solvents over one or more heterogeneous metal based catalyst systems having a reduced catalytic activity for a period of greater than 1 hour, for example 1 to 24 hours, at a temperature in the range of from 50° C to 500° C; wherein said one or more heterogeneous metal based catalyst systems have a yield of less than 90 percent based on the molar conversion of cyanoaldehydes to diamines; and (2) thereby improving the catalytic activity of said one or more heterogeneous metal based catalyst systems.

Further described is a process for improving catalytic activity of catalyst systems for reductive amination of aliphatic cyanoaldehydes to aliphatic diamines comprising the steps of: (1) feeding hydrogen and optionally one or more solvents over one or more heterogeneous metal based catalyst systems having a reduced catalytic activity for a period a period of greater than 1 hour, for example 1 to 24 hours, at a temperature in the range of from 100° C to 500° C; wherein said one or more heterogeneous metal based catalyst systems have a yield of less than 90 percent based on the molar conversion of cyanoaldehydes to diamines; and (2) thereby improving the catalytic activity of said one or more heterogeneous metal based catalyst systems.

In one embodiment, the instant invention provides a process for improving catalytic activity of catalyst systems for reductive amination of aliphatic cyanoaldehydes to aliphatic diamines comprising the steps of: (1) feeding ammonia, optionally hydrogen, and one or more solvents over one or more heterogeneous metal based catalyst systems having a reduced catalytic activity for a period of greater than 1 hour, for example 1 to 24 hours, at a temperature in the range of from 50° C to 500° C; wherein said one or more heterogeneous metal based catalyst systems have a yield of less than 90 percent based on the molar conversion of cyanoaldehydes to diamines; (2) subsequently feeding hydrogen and optionally one or more solvents over said one or more treated heterogeneous metal based catalyst systems with ammonia and hydrogen for a period of greater than 1 hour at a temperature in the range of from 100 ° C to 500° C; and (3) thereby improving the catalytic activity of said one or more heterogeneous metal based catalyst systems.

One or more heterogeneous metal based catalyst systems, obtained according the present invention, having improved catalytic activity may be used to produce one or more cycloaliphatic diamines.

One or more cycloaliphatic diamines may comprise the reaction product of one or more cycloaliphatic cyanoaldehydes selected from the group consisting of 1,3-cyanocyclohexane carboxaldehyde, 1,4-cyanocyclohexane carboxaldehyde, mixtures thereof, and combinations thereof, hydrogen, and ammonia fed into a reductive amination reactor system; wherein said one or more cycloaliphatic cyanoaldehydes, hydrogen, and ammonia are contacted with each other in the presence of one or more heterogeneous metal based catalyst systems having improved catalytic activity at a temperature in the range of from 80° C to about 160° C and a pressure in the range of from 700 to 3500 psig; wherein said one or more cycloaliphatic diamines are selected from the group consisting of 1,3-bis(aminomethyl)cyclohexane, 1,4-bis(aminomethyl)cyclohexane, combinations thereof, and mixtures thereof.

The process for producing cycloaliphatic diamines described herein comprises the steps of: (1) feeding one or more cycloaliphatic cyanoaldehydes selected from the group consisting of 1,3-cyanocyclohexane carboxaldehyde, 1,4-cyanocyclohexane carboxaldehyde, mixtures thereof, and combinations thereof, hydrogen, and ammonia into a reductive amination reactor system; (2) contacting said one or more cycloaliphatic cyanoaldehydes, hydrogen, and ammonia with each other in the presence of one or more heterogeneous metal based catalyst systems having improved catalytic activity at a temperature in the range of from 80° C to about 160° C and a pressure in the range of from 700 to 3500 psig; and (3) thereby forming one or more cycloaliphatic diamines, wherein said one or more cycloaliphatic diamines are diamines selected from the group consisting of 1,3-bis(aminomethyl)cyclohexane, 1,4-bis(aminomethyl)cyclohexane, combinations thereof, and mixtures thereof.

The one or more cycloaliphatic cyanoaldehydes may be selected from the group consisting of 3-cyanocyclohexane carboxaldehyde, 4-cyanocyclohexane carboxaldehyde, mixtures thereof, and combinations thereof.

3-cyanocyclohexane carboxaldehyde, CAS No. 50738-61-9, may have the following structure and formula:

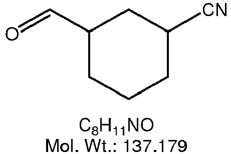

4-cyanocyclohexane carboxaldehyde, CAS No. 18214-33-0, may have the following structure and formula:

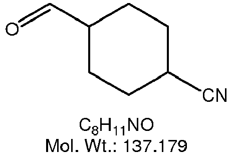

The reaction between one or more cycloaliphatic cyanoaldehydes, hydrogen, and ammonia may take place in the presence of one or more heterogeneous metal based catalyst systems having improved catalytic activity at a temperature in the range of from 60° C to 200° C, for example from 80° C to about 160° C or from 90° C to about 130° C, and a pressure in the range of from 500 to 5000 psig, for example from 700 to 3500 psig or from 1400 to 2500 psig. Such one or more heterogeneous metal based catalyst systems may comprise a metal selected from the group consisting of Co, Ni, Ru, Fe, Cu, Re, Pd, oxides thereof, mixtures thereof, and combinations thereof. Such one or more heterogeneous metal based catalyst systems may comprise a bulk metal catalyst system, sponge-metal catalyst system, supported metal catalyst system, mixtures thereof, or combinations thereof. Such one or more heterogeneous metal based catalyst systems may comprise a bulk Co based catalyst system. In a continuous process, the catalyst lifetime facilitates a weight ratio of the one or more cycloaliphatic diamines to one or more heterogeneous metal based catalyst systems that is greater than 300; for example, greater than 500; or in the alternative greater than 900; or in the alternative greater than 1000. The one or more heterogeneous metal based catalyst systems may further comprise a sponge-metal catalyst. The one or more heterogeneous metal based catalyst systems may further comprise one or more promoters or one or more binding agents. Such one or more promoters may be selected from the group consisting of alkali metals and alkaline earth metals. Such one or more binding agents may comprise silicon oxide, aluminum oxide, titanium oxide, zirconium oxide, mixtures thereof, or combinations thereof. Such one or more heterogeneous metal based catalyst systems are commercially available as Raney Cobalt Catalyst from Grace Davison Catalyst Company, Co-0179T cobalt catalyst from BASF, and Co-138E catalyst from BASF.

In a continuous process, the cost of the catalyst depends on its lifetime, which is equivalent to the weight of product produced per pound of catalyst required. An adequately long lifetime is required for an economically viable continuous process. The one or more heterogeneous metal based catalyst systems may be present in an amount necessary to catalyze the reaction between the one or more cycloaliphatic cyanoaldehydes, hydrogen, and ammonia. For example, the catalyst lifetime facilitates a weight ratio of the cycloaliphatic diamines to the one or more heterogeneous metal based catalyst systems to be greater than 300, for example, greater than 500; or in the alternative, greater than 900; or in the alternative, greater than 1000. In one embodiment, the one or more heterogeneous metal based catalyst systems may, for example, comprise a continuous fixed bed catalyst system.

The one or more heterogeneous metal based catalyst systems may be present in an amount necessary to catalyze the reaction between the one or more cycloaliphatic cyanoaldehydes, hydrogen, and ammonia. The space velocity, which is defined as mass of one or more cycloaliphatic cyanoaldehydes mixture per mass of catalyst per hour, is in the range of from 0.1 to 10.0 per hour; for example, from 0.1 to 5.0 per hour; or in the alternative, from 0.1 to 3.0 per hour; or in the alternative, from 0.1 to 2.0 per hour; or in the alternative, from 0.1 to 1.0 per hour; or in the alternative, from 0.3 to 0.8 per hour.

Ammonia is present in excess amount relative to the one or more cycloaliphatic cyanoaldehydes. Ammonia may, for example, be present in a range of 2 to 50 moles per mole of one or more cycloaliphatic cyanoaldehydes; or in the alternative, in a range of 5 to 40 moles per mole of one or more cycloaliphatic cyanoaldehydes; or in the alternative, in a range of 8 to 30 moles per mole of one or more cycloaliphatic cyanoaldehydes. Hydrogen may, for example, be present in a range of 3 to 30 moles per mole of one or more cycloaliphatic cyanoaldehydes; or in the alternative, in a range of 3 to 10 moles per mole of one or more cycloaliphatic cyanoaldehydes; or in the alternative, in a range of 3 to 6 moles per mole of one or more cycloaliphatic cyanoaldehydes.

The reaction between one or more cycloaliphatic cyanoaldehydes, hydrogen, and ammonia may optionally take place in the presence of one or more solvents. Such solvents include, but are not limited to, water; 2-propanol (isopropylalcohol), CAS No. 67-63-0; methanol, CAS No. 67-56-1; t-butanol, CAS No. 75-65-0; and tetrahydrofuran (THF), CAS No. 109-99-9. The feed into the reactor may comprise 0 to 90 percent by weight of one or more solvents, based on the combined weight of one or more cycloaliphatic cyanoaldehydes and the one or more solvents; or in the alternative, 0 to 30 percent by weight of one or more solvents, based on the combined weight of one or more cycloaliphatic cyanoaldehydes and the one or more solvents; or in the alternative, 0 to 10 percent by weight of one or more solvents, based on the combined weight of one or more cycloaliphatic cyanoaldehydes and the one or more solvents.

The reaction between one or more cycloaliphatic cyanoaldehydes, hydrogen, and ammonia may take place in a continuous reductive amination reactor system; or in the alternative, it may take place.in a batch reactor system; or in the alternative, it may take place in a semi-batch reactor system. Such reactor systems are generally known to a person of ordinary skill in the art. The continuous reductive amination reactor system, the semi-batch reductive amination reactor system, or the batch reductive amination reactor system may comprise one or more reactors in series, in parallel, or combinations thereof.

The one or more cycloaliphatic diamines produced as described herein may be selected from the group consisting of 1,3-bis(aminomethyl)cyclohexane, 1,4-bis(aminomethyl)cyclohexane; combinations thereof, and mixtures thereof.

1,3-bis(aminomethyl)cyclohexane, CAS No. 2579-20-6, may have the following structure or formula:

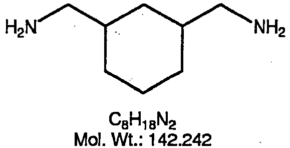

1,4-bis(aminomethyl)cyclohexane, CAS No. 2549-93-1, may have the following structure or formula:

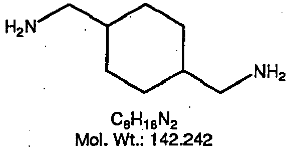

Additional byproducts may include 3-(aminomethyl)-cyclohexanecarbonitrile, CAS No. 23083-50-3; 4-(aminomethyl)-cyclohexanecarbonitrile, CAS No. 54898-73-6; 3-azabicyclo[3.3.1]nonane, CAS No. 280-70-6; 3-azabicyclo[3.3.1]non-2-ene, CAS No. 7129-32-0; 7-amino-bicyclo[2.2.1]heptane-1-methanamine; 3-(aminomethyl)-cyclohexanemethanol, CAS No. 925921-54-6; 4-(aminomethyl)-cyclohexanemethanol, CAS No. 1074-62-0.

In a process for producing cycloaliphatic diamines, one or more cycloaliphatic cyanoaldehydes, hydrogen, ammonia, and optionally one or more solvents are introduced into a reductive amination reactor system and reacted with each other in the presence of one or more heterogeneous metal based catalyst systems having improved catalytic activity at a temperature in the range of from 80° C to about 160° C and a pressure in the range of from 700 to 3500 psig to yield one or more cycloaliphatic diamines.

Further described is that one or more cycloaliphatic cyanoaldehydes are contacted with ammonia first and then the product mixture including the product of the reaction of one or more cycloaliphatic cyanoaldehydes with ammonia is contacted with hydrogen in the presence of one or more heterogeneous metal based catalyst systems having improved catalytic activity.

A product mixture including one or more aliphatic diamines, optionally a portion of the product of the reaction of one or more cycloaliphatic cyanoaldehydes with ammonia, optionally, a portion of the ammonia, optionally a portion of the hydrogen, optionally a portion of one or more by-products, optionally a portion of the water, and optionally a portion of the one or more solvents is formed in the one or more reactor systems, as described hereinabove. The product mixture is then removed from the one or more reactor systems and transferred to one or more distillation columns arranged in sequential order. After the product mixture is transferred to one or more distillation columns arranged in sequential order, at least a portion of the ammonia, a portion of the hydrogen, or a mixture thereof is removed from the product mixture via one or more distillation steps. Subsequently, at least a portion of the one or more solvents, if optionally present, and/or water is removed via one or more distillation steps. Subsequently, at least a portion of the product of the reaction of one or more cycloaliphatic cyanoaldehydes with ammonia or one or more by-products is removed via one or more distillation steps, thus separating the one or more aliphatic diamines from the product mixture and converting the one or more cyanoaldehydes to one or more aliphatic diamines. The distillation process is further described in the U.S. provisional patent application with serial No. 61/230,300.

The one or more cycloaliphatic diamines produced as described herein may be used as a precursor to an aliphatic diisocyanate (bis(isocyanatomethyl)cyclohexane), as a chain extender in certain polyurethanes systems, or as an epoxy curing agent.

### Examples

The following examples illustrate the present invention but are not intended to limit the scope of the invention, which is defined by the claims.

### Inventive Example 1

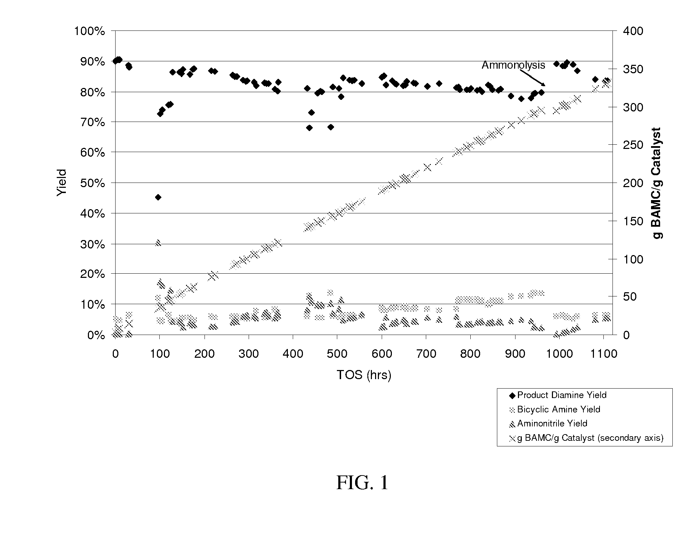

A reactor feed mixture was prepared by combining crude cyanoaldehyde, wherein the crude cyanoaldehyde comprised 85 to 90 percent by weight of cyanoaldehyde, with tert-butanol (t-butanol) to give a crude cyanoaldehyde concentration of approximately 70 weight percent. The mixture was passed through a bed of sodium carbonate. A reactor tube was loaded with 7.0 g of BASF Co-0179T catalyst, and diluted with 100 grit (150 µm) SiC (silicon carbide) fines. The temperature of the reactor was raised to 250° C The temperature of the reactor was maintained for 14 hours at 250° C, and then it was reduced to a temperature in the range of approximately less than 80° C under continued hydrogen flow. As soon as the reactor temperature reached below 80° C, an excess flow of ammonia (at about 3 to 5 times the desired set point, as described below) was introduced into the reactor. The temperature and pressure were then changed to the desired set points of approximately 100° C and approximately 1400 psig. After at least one hour, the ammonia rate was decreased to the desired set point of 0.21 mL/min with an organic feed rate of 0.06 mL/min, a liquid hourly space velocity (LHSV, based on combined organic and ammonia mass feed rates, (mL Feed/g catalyst/hr)) of 2.3 mL/g/hr with a hydrogen to cyanoaldehyde molar ratio of 5. Samples were periodically collected from the reactor and analyzed by gas chromatography. The reactor was run for 960 hours time on stream (TOS) and the temperature was increased to 120°C to maintain low production of aminonitrile. The product diamine (1,3- and 1,4-bis(aminomethyl)cyclohexane) yield had been reduced from 90.3 percent to 79.7 percent due to loss of catalyst activity. An ammonolysis was performed at 960 hours TOS. Ammonia at 0.5 mL/min and hydrogen at 50 sccm were fed over the solid cobalt catalyst at 145° C and 1400 psi for 8 hours. The ammonia flow was stopped, and hydrogen was increased to 100 sccm over the solid cobalt catalyst at 250° C and 1400 psi for 13 hours. After reducing the reactor temperature to <80°C under continued hydrogen flow, an excess flow of ammonia (at least 3-5 times desired set point) was initiated. Reaction conditions were then reestablished with an LHSV of 2.3 mL/g/hr and a hydrogen to cyanoaldehyde molar ratio of 5. The temperature was set at 100° C with pressure at 1400 psi. After the ammonolysis, i.e. treating a solid catalyst with ammonia and/or hydrogen at elevated temperatures, exemplary range of 140° C to 300° C, the product diamine yield had returned to 88.3 percent, as shown in Fig. 1. The deactivation of the catalyst resumed after the ammonolysis.

### Inventive Example 2

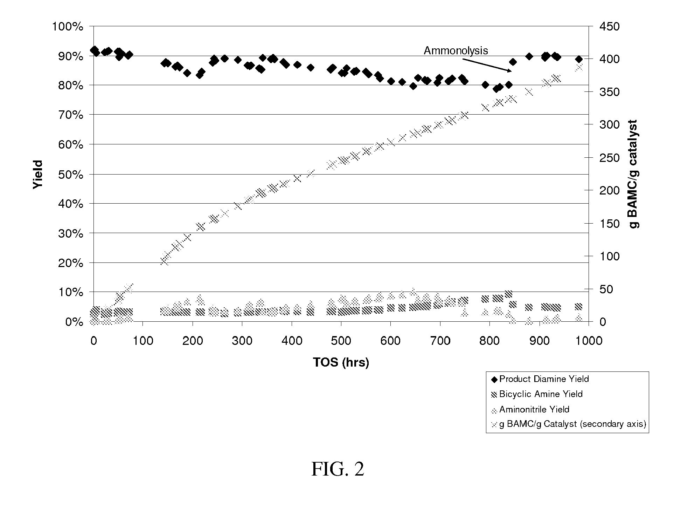

A reactor feed mixture was prepared by combining crude cyanoaldehyde, wherein the crude cyanoaldehyde comprised 85 to 90 percent by weight of cyanoaldehyde, with tert-butanol (t-butanol) to give a crude cyanoaldehyde concentration of approximately 70 weight percent. The mixture was passed through a bed of sodium carbonate. A reactor tube was loaded with 7.0 g of BASF Co-0179T catalyst, and diluted with 100 grit (150 µm) SiC (silicon carbide) fines. The temperature of the reactor was raised to 233° C. The temperature of the reactor was maintained for 14 hours at 233° C, and then it was reduced to a temperature in the range of approximately less than 80° C under continued hydrogen flow. As soon as the reactor temperature reached below 80° C, an excess flow of ammonia (at about 3 to 5 times the desired set point, as described below) was introduced into the reactor. The temperature and pressure were then changed to the desired set points of approximately 100° C and approximately 2000 psig. After at least one hour, the ammonia rate was decreased to the desired set point of 0.22 mL/min with an organic feed rate of 0.07 mL/min, a weight hourly space velocity (WHSV, based on combined organic and ammonia mass feed rates) of 2.8 hr⁻¹ with a hydrogen to cyanoaldehyde molar ratio of 5. Samples collected between 0 and 30 hours time on stream showed 91-92 percent molar yield of diamine (1,3- and 1,4-bis(aminomethyl)cyclohexanes), with 0-0.25 percent molar yield of aminonitrile and 2.3-3.6 percent molar yield of bicyclic amine (3-azabicyclo[3.3.1]nonane), as shown in Fig. 2. After 50 hours of time on stream (TOS), the flow rates were increased for a combined WHSV of 4 hr⁻¹. Productivity at the higher rate was 1.23 x 10⁻⁷ moles diamine/g-catalyst/s. The reactor temperature was increased in 5 degree increments to maintain low production of aminonitrile. At 840 hours TOS, the reactor temperature was approximately 120° C and pressure was approximately 2000 psig. At 840 hours TOS, the reactor effluent provided a yield of 80 percent to product diamine, 9.1 percent to bicyclic amine, and 2.6 percent to aminonitrile. An ammonolysis, i.e. treating a solid catalyst with ammonia and/or hydrogen at elevated temperatures, exemplary range of 140° C to 300° C, was performed at 840 hours TOS. Ammonia at 0.5 mL/min and hydrogen gas at 50 sccm (standard cubic centimeters per minute) were fed over the solid cobalt catalyst at 145° C and 1900 psi for 8 hours. The ammonia flow was stopped, and hydrogen was increased to 100 sccm over the solid cobalt catalyst at 250° C and 1900 psi for 13 hours. After reducing the reactor temperature to approximately less than 80°C under continued hydrogen flow, an excess flow of ammonia (at least 3-5 times desired set point) was introduced. Reaction conditions were then reestablished with an LHSV of 2.1 mL/g/hr and a hydrogen to cyanoaldehyde molar ratio of 5. The organic feed brought on-stream after the ammonolysis was different in that it was treated with Li₂CO₃ and had 8.8 percent water content. The temperature was set at 100° C with pressure at 2000 psi. After the ammonolysis, the product diamine yield had returned to 89.7 percent, as shown in Fig. 2, with a productivity of 6.2 x 10⁻⁷ moles diamine/g-catalyst/s.

## Claims

1. A process for improving catalytic activity of catalyst systems for reductive amination of aliphatic cyanoaldehydes to aliphatic diamines comprising the steps of:
feeding ammonia, optionally hydrogen, and one or more solvents over one or more heterogeneous metal based catalyst systems having a reduced catalytic activity for a period of greater than 1 hour at a temperature in the range of from 50°C to 500°C; wherein said one or more heterogeneous metal based catalyst systems have a yield of less than 90 percent based on the molar conversion of cyanoaldehydes to diamines;
subsequently feeding hydrogen and optionally one or more solvents over said one or more heterogeneous metal based catalyst systems for a period of greater than 1 hour at a temperature in the range of from 100°C to 500°C; and
thereby improving the catalytic activity of said one or more heterogeneous metal based catalyst systems.

2. One or more heterogeneous metal based catalyst systems having improved catalytic activity obtained via the method of claim 1.

## Patentansprüche

1. Ein Verfahren zum Verbessern der katalytischen Aktivität von Katalysatorsystemen für die reduktive Aminierung von aliphatischen Cyanoaldehyden zu aliphatischen Diaminen, das die folgenden Schritte beinhaltet:
Speisen von Ammoniak, optional Wasserstoff und einem oder mehreren Lösungsmitteln über ein oder mehrere heterogene metallbasierte Katalysatorsysteme mit einer reduzierten katalytischen Aktivität für einen Zeitraum von mehr als 1 Stunde bei einer Temperatur in dem Bereich von 50 °C bis 500 °C; wobei das eine oder die mehreren heterogenen metallbasierten Katalysatorsysteme eine Ausbeute von weniger als 90 Prozent auf der Basis der molaren Umwandlung von Cyanoaldehyden zu Diaminen aufweisen;
anschließend Speisen von Wasserstoff und optional einem oder mehreren Lösungsmitteln über das eine oder die mehreren heterogenen metallbasierten Katalysatorsysteme für einen Zeitraum von mehr als 1 Stunde bei einer Temperatur in dem Bereich von 100 °C bis 500 °C; und
dadurch Verbessern der katalytischen Aktivität des einen oder der mehreren heterogenen metallbasierten Katalysatorsysteme.

2. Ein oder mehrere heterogene metallbasierte Katalysatorsysteme mit einer verbesserten katalytischen Aktivität, erhalten über die Methode gemäß Anspruch 1.

## Revendications

1. Un procédé pour améliorer l'activité catalytique de systèmes de catalyseurs pour l'amination réductrice de cyanoaldéhydes aliphatiques en diamines aliphatiques comprenant les étapes consistant à :
amener de l'ammoniac, facultativement de l'hydrogène, et un ou des solvants sur un ou des systèmes de catalyseurs à base de métaux hétérogènes ayant une activité catalytique réduite pendant une période supérieure à 1 heure à une température comprise dans la gamme allant de 50 °C à 500 °C ; dans lequel ce ou ces dits systèmes de catalyseurs à base de métaux hétérogènes ont un rendement inférieur à 90 pour cent basé sur la conversion molaire de cyanoaldéhydes en diamines ;
amener subséquemment de l'hydrogène et facultativement un ou des solvants sur ce ou ces dits systèmes de catalyseurs à base de métaux hétérogènes pendant une période supérieure à 1 heure à une température comprise dans la gamme allant de 100 °C à 500 °C ; et
améliorer de ce fait l'activité catalytique de ce ou ces dits systèmes de catalyseurs à base de métaux hétérogènes.

2. Un ou des systèmes de catalyseurs à base de métaux hétérogènes ayant une activité catalytique améliorée obtenue via la méthode de la revendication 1.
